# EUROPÄISCHE PATENTSCHRIFT

(11) **EP 1 044 654 B1**
(45) Veröffentlichungstag und Bekanntmachung des Hinweises auf die Patenterteilung: **31.03.2004**
(21) Anmeldenummer: 00250244.1
(22) Anmeldetag: 07.11.1996
(51) Int. Cl.: A61B 18/14

(54) **Anordnung zur elektro-thermischen Behandlung des menschlichen oder tierischen Körpers**
Arrangement for electrothermal treatment of the human or animal body
Système de traitement électrothermique du corps humain ou animal

(30) Priorität: 08.11.1995 DE 19541566
(43) Veröffentlichungstag der Anmeldung: 18.10.2000
(62) Teilanmeldung aus: 96945879.3
(73) Patentinhaber: Desinger, Kai, Dr., 12157 Berlin (DE)
(72) Erfinder: Desinger, Kai, 12157 Berlin (DE); Müller, Gerhard, 14129 Berlin (DE)
(74) Vertreter: Eisenführ, Speiser & Partner

(56) Entgegenhaltungen:
- WO-A-92/19167
- WO-A-94/02077
- WO-A-95/10320
- DE-A- 3 930 451
- US-A- 4 565 200
- US-A- 4 832 048
- US-A- 5 007 908

## Beschreibung

Die Erfindung betrifft eine Anordnung zur elektrothermischen Behandlung des menschlichen oder tierischen Körpers, insbesondere zur Elektrokoagulation oder Elektrotomie, gemäß dem Oberbegriff des Anspruchs 1.

Eine derartige anordnung ist aus US-A-4 565 200 bekannt.

Es ist seit längerem in der Chirurgie bekannt, zur Gewebekoagulation und zur Gewebetrennung hochfrequente Wechselströme im Frequenzbereich von 300 kHz bis 2 MHz zu verwenden, wodurch das behandelte Gewebe koaguliert bzw. vaporisiert wird, was als Elektrokoagulation bzw. Elektrotomie bezeichnet wird. Hierbei ist zu unterscheiden zwischen der monopolaren und der bipolaren HF-Thermotherapie.

Bei der monopolaren HF-Thermotherapie wird eine Elektrode - auch als Neutralelektrode bezeichnet - als großflächige Patientenableitung ausgelegt und in der Nähe der Eingriffsstelle am Patienten angebracht. Die eigentliche Arbeitselektrode - auch als Aktivelektrode bezeichnet - ist in ihrer Form an die jeweilige Anwendung angepaßt. So werden zur Gewebekoagulation großflächige Kugel-, Platten- oder Nadelelektroden verwendet, während bei der Gewebetrennung dünne Lanzetten- oder Schlingenelektroden eingesetzt werden.

Bei der bipolaren HF-Thermotherapie hingegen sind beide Elektroden in unmittelbarer Nähe zur Eingriffsstelle angeordnet, so daß die Wirkung des Wechselstroms auf die unmittelbare Umgebung der Eingriffsstelle begrenzt ist, wodurch ein hohes Maß an Sicherheit für Patient und Anwender gegeben ist, da Unfälle durch kapazitive Leckströme oder Verbrennung an der Neutralelektrode nicht mehr auftreten können. Ein weiterer Vorteil der bipolaren HF-Thermotherapie besteht in dem wesentlich geringeren Lastwiderstand des Gewebes zwischen den beiden Elektroden, was unter Beibehaltung des thermischen Effekts eine Verringerung der erforderlichen Generatorleistung ermöglicht.

Die HF-Thermotherapie läßt sich weiterhin einteilen nach der Lage der Elektroden in die Oberflächenkoagulation einerseits und die Tiefenkoagulation andererseits.

Bei der Oberflächenkoagulation werden in der bipolaren Technik zwei parallel angeordnete Tastelektroden verwendet, die auf die Gewebeoberfläche aufgesetzt werden, wodurch das darunterliegende Gewebe infolge des Stromflusses erhitzt und damit koaguliert wird.

Bei der Tiefenkoagulation ist es zur monopolaren Elektrotomie bekannt, Nadel-, Lanzetten- oder Schlingenelektroden zu verwenden. Hierbei müssen an der Aktivelektrode elektrische Lichtbögen generiert werden, um das vor der Aktivelektrode liegende Gewebe zu vaporisieren und damit einen Gewebsschnitt zu realisieren. Bei der monopolaren Technik ist dies verhältnismäßig einfach, da es hier nur einer bestimmten Feldstärke bedarf, um an der Aktivelektrode einen Funkenüberschlag auszulösen. Die bipolare Technik stellt hier größere Anforderungen an die Konzeption der Elektrodenkonfiguration, da die physikalischen Vorgänge hierbei nicht einfach zu beherrschen sind. Es sind deshalb nur wenige bipolare Elektrodenanordnungen für die Tiefenkoagulation bekannt, so beispielsweise die bipolare Nadelelektrode, die sich unter anderem zur Myomtherapie eignet. Diese vorbekannte bipolare Elektrodenanordnung besteht aus zwei parallel angeordneten Nadelelektroden, die in das Gewebe eingestochen werden, wodurch das zwischen den Elektroden liegende Gewebe infolge des Stromflusses erhitzt und damit koaguliert wird.

Nachteilig bei den bekannten bipolaren Elektrodenanordnungen zur Tiefenkoagulation ist jedoch die relativ aufwendige Plazierung der Elektroden durch zwei Einstichstellen. Darüber hinaus läßt sich die Feldverteilung vom Anwender nur relativ ungenau vorherbestimmen, da die relative Lage der beiden Elektroden zueinander in der Regel nicht exakt vorgegeben werden kann.

Es ist aus DE 43 22 955 A1 weiterhin bekannt, zur Koagulation von Körpergewebe Laserstrahlung zu verwenden, die über einen zylindrischen Lichtwellenleiter ins Therapiegebiet übertragen werden kann, wobei der vorbekannte Lichtwellenleiter zusätzlich auch die Übertragung von Ultraschallenergie ermöglicht, so daß die beiden Therapieverfahren der Ultraschall-Gewebetrennung und der Laser-Koagulation kombiniert werden können.

Aus DE 44 32 666 A1 ist weiterhin ein Wellenleiter bekannt, der neben der Übertragung von Ultraschallwellen und Laserstrahlung auch die Übertragung von Hochfrequenzenergie ermöglicht, um gleichzeitig zur Laser- und Ultraschallchirurgie auch die eingangs erwähnten Verfahren der Hochfrequenzchirurgie anwenden zu können. Hierzu ist der vorbekannte Wellenleiter zylindrisch ausgeführt und weist zur Übertragung der Hochfrequenzenergie zwei ebenfalls zylindrische Schichten aus elektrisch leitfähigem Material auf, die elektrisch gegeneinander isoliert sind. Der vorbekannte Wellenleiter ermöglicht somit zwar die Übertragung von Hochfrequenzenergie von einem extrakorporal angeordneten Hochfrequenzgenerator ins Therapiegebiet, gestattet jedoch keine Abgabe der Hochfrequenzenergie an das Körpergewebe.

Aus dem US Patent 4,565,200 ist ein Katheter bekannt, der zwei relativ zueinander verschiebbare, koaxiale Elektroden aufweist, die ausweislich der Beschreibung dem Einleitung von Hochfrequenzenergie in Körpergewebe zum Erzeugen von Läsionen dienen sollen.

Aus dem US Patent 5,007,908 ist es bekannt, in eine Koagulationssonde Flüssigkeit einzuspülen und am distalen Ende austreten zu lassen, um den Behandlungsort zu spülen und die endoskopische Betrachtung des Behandlungsortes zu erleichtern.

Der Erfindung liegt die Aufgabe zugrunde, eine Anordnung zur elektrothermischen Behandlung des menschlichen oder tierischen Körpers zu schaffen, die mittels einer bipolaren Elektrodenanordnung eine interstitielle Gewebekoagulation ermöglicht und die vorstehend genannten Nachteile der bekannten Anordnungen dieser Art vermeidet.

Die Aufgabe wird, ausgehend von einer Anordnung gemäß dem Oberbegriff des Anspruchs 1 oder 4, durch die im kennzeichnenden Teil des Anspruchs 1 bzw. 4 angegebenen Merkmale gelöst.

Die Erfindung schließt die technische Lehre ein, zur Thermotherapie eine bipolare Elektrodenanordnung zu verwenden, bei der die beiden Elektroden hintereinander auf einem langgestreckten Katheter angeordnet sind, um eine gemeinsame Einführung der beiden Elektroden in den Körper durch eine einzige Einstichstelle zu ermöglichen, wobei die beiden Elektroden mit dem Katheter verbunden oder ein Bestandteil des Katheters sind.

Der Begriff Katheter ist hierbei und im folgenden allgemein zu verstehen und nicht auf die vorzugsweise verwendeten und im folgenden ausführlich beschriebenen hohlzylindrischen Anordnungen beschränkt, sondern auch mit massiven Anordnungen nahezu beliebiger Querschnitte realisierbar. Maßgebend für die erfindungsgemäße Funktion ist lediglich, dass die beiden Elektroden gemeinsam durch eine Einstichstelle in den Körper des Patienten eingeführt werden können.

Durch den Katheter ist es möglich, die Elektroden in tiefen Gewebsschichten zu plazieren und dort eine partielle Gewebekoagulation zu erreichen.

Die Elektroden sind bei der erfindungsgemäßen Anordnung für die Zuführung der zur Gewebeerwärmung erforderlichen elektrischen Energie mit einer Stromquelle verbunden, wobei der Begriff Stromquelle nicht auf Stromquellen im engeren Sinne mit einem konstanten Strom beschränkt ist, sondern auch die vorzugsweise verwendeten Wechselstromgeneratoren, insbesondere Hochfrequenzgeneratoren, einschließt.

Gemäß der Erfindung ist der axiale Abstand zwischen den beiden Elektroden einstellbar, um die Feldverteilung variieren zu können. Beträgt die isolatorlänge zwischen den beiden Elektroden in axialer Richtung beispielsweise weniger als das zweifache des Elektrodendurchmessers, so lassen sich vorteilhaft kugelförmige Koagulationsnekrosen erzielen, wohingegen die Form der Koagulationsnekrosen bei größeren Isolatorlängen eher oval ist.

Es ist vorgesehen, die proximale Elektrode mindestens an ihrem distal gelegenen Ende hohl auszuführen, um die distale Elektrode in ihrem inneren aufnehmen zu können. Um hierbei eine axiale Verschiebbarkeit der distalen Elektrode in der proximalen Elektrode zu ermöglichen ist der Außenquerschnitt der distalen Elektrode kleiner als der Innenquerschnitt der proximalen Elektrode. Hierbei ist es wichtig, die beiden Elektroden in geeigneter Weise elektrisch gegen einander zu isolieren, da die beiden Elektroden in axiale Richtung überlappen können. Hierzu ist auf der Innenseite der proximalen Elektrode oder auf der Außenseite der distalen Elektrode eine elektrische Isolation vorgesehen, die beispielsweise - wie in der eingangs angeführten Druckschrift DE 44 32 666 Al - aus einer dielektrischen Beschichtung oder einer Isolierstoffhülse bestehen kann, die vorzugsweise aus PTFE oder Polymid besteht, wobei der Querschnitt der Isolierstoffhülse vorzugsweise derart an den Querschnitt der distalen bzw. proximalen Elektrode angepasst ist, dass die Isolierstoffhülse mit der proximalen bzw. distalen Elektrode eine Presspassung bildet und somit auf der Elektrode fixiert ist. Die beiden Elektroden sind also koaxial angeordnet und in axialer Richtung gegeneinander verschiebbar, um die Feldverteilung ändern zu können, wobei die distale Elektrode auf einem Teil ihrer Längserstreckung von der proximalen Elektrode aufgenommen wird.

Der Querschnitt der beiden Elektroden ist in dieser Variante der Erfindung wie auch den anderen Erfindungsvarianten vorzugsweise zylindrisch, wobei der Innendurchmesser der proximalen Elektrode in dieser Variante größer sein muss als der Außendurchmesser der distalen Elektrode, damit die distale Elektrode in axialer Richtung verschiebbar ist. Die Erfindung ist jedoch nicht auf zylindrische Elektrodenformen beschränkt, sondern auch mit anderen Elektrodenquerschnitten realisierbar. Maßgebend für die erfindungsgemäße Funktion ist in dieser Variante der Erfindung lediglich, dass der Innenquerschnitt der proximalen Elektrode derart an den Außenquerschnitt der proximalen Elektrode angepasst ist, dass die distale Elektrode in der proximalen Elektrode in axialer Richtung verschoben werden kann, um den axialen Abstand zwischen den beiden Elektroden verändern zu können.

In einer anderen Variante der Erfindung wird die Einstellung des axialen Abstandes zwischen den beiden Elektroden durch ein langgestrecktes Trägerelement aus elektrisch isolierendem Material ermöglicht, das in der proximalen Elektrode verschiebbar angeordnet ist und an seinem distalen Bereich seitlich die distale Elektrode aufweist. Die proximale Elektrode ist deshalb zumindest an ihrem distalen Ende hohl ausgeführt, um das Trägerelement aufnehmen zu können. Der Innenquerschnitt der proximalen Elektrode ist hierbei derart an den Außenquerschnitt des Trägerelements angepasst, dass das Trägerelement in axialer Richtung verschiebbar ist, um den axialen Abstand zwischen dem distalen Ende der proximalen Elektrode und der auf der Trägerelement angeordneten distalen Elektrode einstellen zu können. Die distale Elektrode ist in dieser Variante der Erfindung seitlich an dem elektrisch isolierenden Trägerelement angebracht und kann beispielsweise aus einer ringförmigen metallischen Beschichturig oder einer metallischen Hülse bestehen, die zur Montage axial auf das Trägerelement aufgeschoben wird und mit diesem eine Presspassung bildet.

Gemäß einer weiterbildenden Variante der Erfindung sind mehr als zwei Elektroden vorgesehen, die in axialer Richtung des Katheters zueinander beabstandet angeordnet sind.
Die Elektroden können beispielsweise - wie bereits vorstehend beschrieben - seitlich an einem langgestreckten Trägerelement angebracht sein oder - wie bereits zuvor erläutert - jeweils durch ein Verbindungselement aus elektrisch isolierendem Material voneinander getrennt werden.

In der bevorzugten Ausführungsform dieser Variante können die einzelnen, entlang der Längsachse des Katheters axial verteilt angeordneten Elektrodenpaare getrennt angesteuert werden und weisen hierzu jeweils getrennte Zuleitungen auf, die vorzugsweise durch einen Hohlkanal im Inneren des Katheters aus dem Körper herausgeführt sind und mit einem entsprechenden Steuergerät verbunden werden können, welches eine individuelle Einstellung beispielsweise von Strom, Spannung und/oder Frequenz ermöglicht. Aufgrund der Überlagerung der von den einzelnen Elektrodenpaaren erzeugten Felder ist es auf diese Weise möglich, die Feldverteilung in weiten Grenzen frei vorzugeben, um beispielsweise bei einer Elektrokoagulation möglichst wenig gesundes Gewebe zu zerstören. In einer vorteilhaften Weiterbildung dieser Variante weist das extrakorporale Steuergerät mehrere Speicherelemente auf, in denen die elektrischen Parameter wie Strom, Spannung und Frequenz für verschiedene Feldverteilungen gespeichert sind, so dass der Benutzer sich lediglich für die gewünschte Feldverteilung entscheiden muss, woraufhin das Steuergerät dann die zur Erreichung dieser Feldverteilung erforderlichen elektrischen Parameter aus dem jeweiligen Speicherelement ausliest und die einzelnen Elektrodenpaare entsprechend ansteuert.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
- Figur 1: eine Anordnung zur elektrothermischen Behandlung mit einem Katheter zur Einführung der Elektroden in den Körper und einem Handhabungsteil zur Führung des Katheters,
- Figur 2a, 2b: den Katheter der Anordnung aus Figur 1 mit eingefahrener bzw. ausgefahrener Kernelektrode,
- Figur 3a, 3b: verschiedene Katheter mit feststehenden Elektroden zur Erreichung einer vorgegebenen Feldverteilung,
- Figur 4: einen Katheter mit fünf Elektroden zur Ermöglichung besonderer Feldverteilungsformen,
- Figur 5: einen flexiblen Katheter zum Einsatz in der minimalinvasiven Chirurgie,
- Figur 6a, 6b, 6c: verschiedene Katheter, die eine Zuführung von Spülflüssigkeit ermöglichen sowie
- Figur 7: einen weiteren Katheter mit einstellbarem Elektrodenabstand.

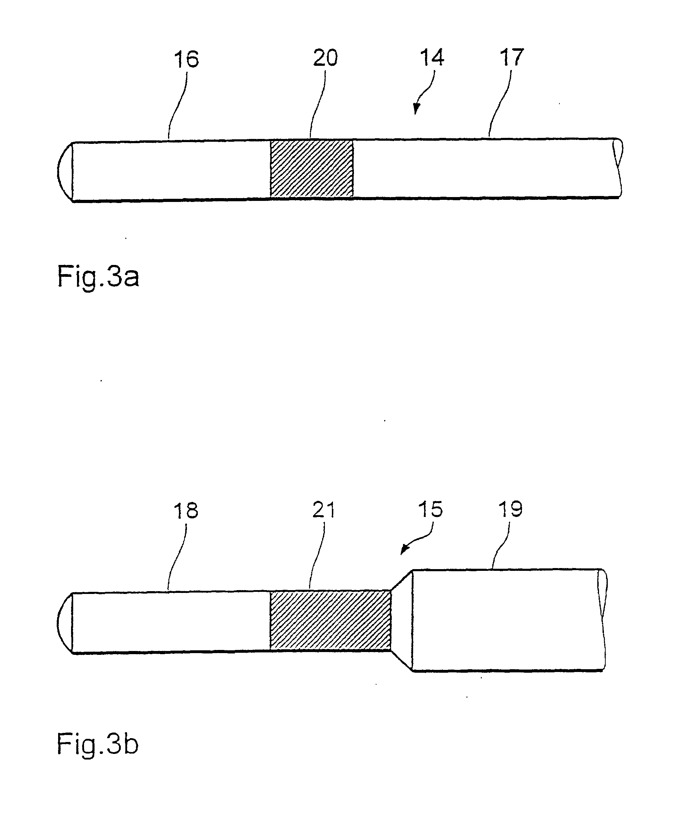

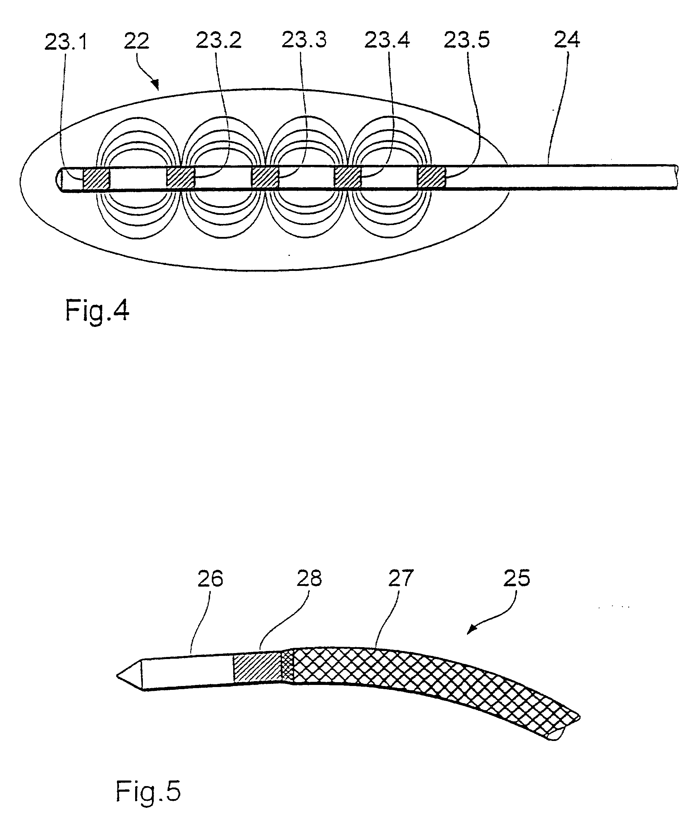

Die ausführungen gemäß Figuren 3 und 4 sind kein Teil der Erfindung.

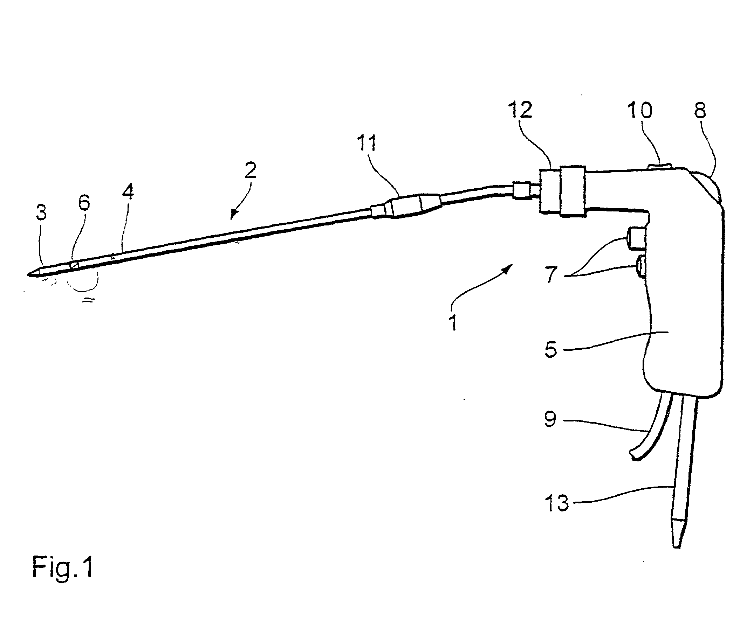

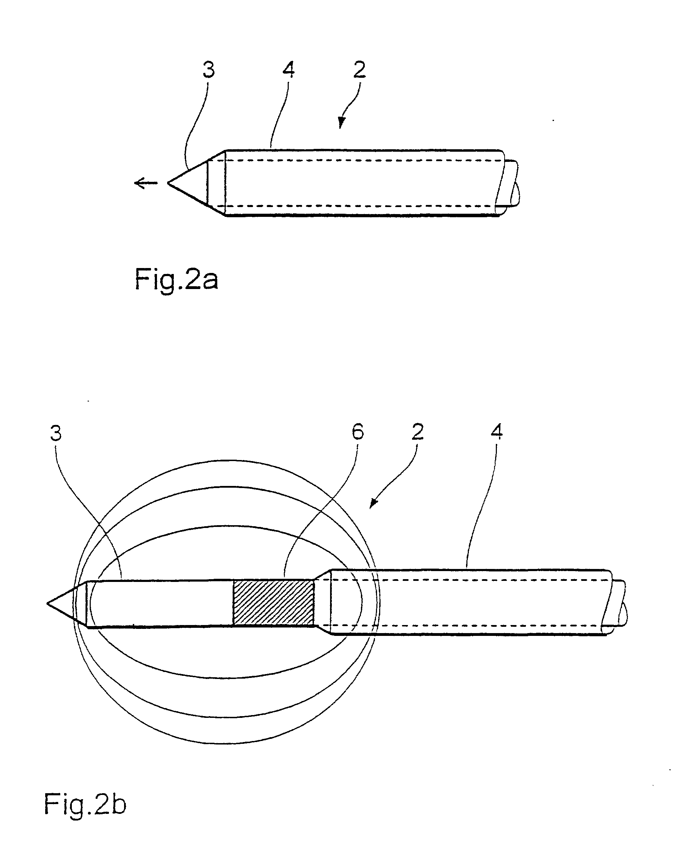

Figur 1 zeigt als bevorzugtes Ausführungsbeispiel der Erfindung eine Anordnung 1 zur elektro-thermischen Behandlung des menschlichen oder tierischen Körpers, die im wesentlichen aus einem Katheter 2 mit einer Kernelektrode 3 und einer Mantelelektrode 4 sowie einem Handhabungsteil 5 zur Führung des Katheters 2 besteht, wobei der Katheter 2 detailliert in den Figuren 2a und 2b dargestellt ist.

Der Katheter 2 ermöglicht eine Einstellung des axialen Abstandes zwischen den beiden Elektroden 3, 4, um die Feldverteilung im Therapiegebiet vorgeben zu können. Hierzu weist der Katheter 2 die zylindrische Kernelektrode 3 aus Edelstahl mit einem Durchmesser von 800 µm auf, die an ihrer Mantelfläche bis auf ihr distales Ende zur elektrischen Isolierung mit einer 50 µm dicken Polyimidschicht 6 versehen ist. Diese beschichtete Kernelektrode 3 liegt koaxial verschiebbar in der hohlzylindrischen Mantelelektrode 4, die ebenfalls aus Edelstahl besteht und einen Innendurchmesser von 900 µm aufweist. Der Außendurchmesser der Mantelelektrode 4 beträgt 1500 µm bei einer Länge von 10 cm.

Durch einen in das Handhabungsteil 5 integrierten Verschiebemechanismus läßt sich die innere Kernelektrode 3 vor dem Einstechen des Katheters 2 in das Gewebe zurückziehen, so daß die Kernelektrode 3 und die Mantelelektrode 4 zusammen einen symmetrisch angeschliffenen Einstichdorn bilden, wie in Figur 2a dargestellt ist.

Nach dem Einführen des Katheters 2 in das Gewebe läßt sich die Kernelektrode 3 dann in axialer Richtung ausfahren und bildet so mit der isolierenden Polyimidschicht 6 und der Mantelelektrode 4 eine Dipolkonfiguration, wie in Figur 2b dargestellt ist. Darüber hinaus ermöglicht die axiale Verschiebung der Kernelektrode 3 auch eine Einstellung des axialen Abstandes zwischen den beiden Elektroden 3, 4 und damit eine gezielte Beeinflussung der Feldverteilung im Therapiegebiet. Die Bedienung des Verschiebemechanismus für die Kernelektrode 3 erfolgt durch eine Druckknopfwippe 7, die in das aus ergonomischen Gründen als Pistolenhandgriff geformte Handhabungsteil 5 integriert ist. Darüber hinaus enthält das Handhabungsteil 5 einen Schalter 8, mit dem die Elektrodenanordnung an einen HF-Generator angeschlossen werden kann, der mit dem Handhabungsteil 5 über eine elektrische Zuleitung 9 verbunden ist. Weiterhin weist das Handhabungsteil 5 einen Entriegelungsmechanismus 10 auf, über den die Kernelektrode 3 freigegeben und anschließend axial aus der Mantelelektrode 4 herausgezogen werden kann. Nach dem Entriegeln eines Verschlußmechanismus' 11 kann dann auch die Mantelelektrode 4 axial herausgezogen werden. Die Trennung der Elektroden 3, 4 von dem Handhabungsteil 5 ermöglicht in einfacher Weise eine Sterilisierung und anschließende Wiederverwertung der Elektroden 3, 4.

Der aus Kern- und Mantelelektrode 3, 4 bestehende Katheter 2 ist über ein drehbares Aufnahmelager 12 mit dem Handhabungsteil 5 verbunden und ermöglicht durch seine abgewinkelte Form ein an das Sichtfeld des Arztes angepaßtes Arbeiten, wie es zum Beispiel bei der Nasenmuschelkoagulation notwendig ist.

Weiterhin ermöglicht die dargestellte Anordnung 1 die Einleitung einer Spülflüssigkeit in das Gewebe im Therapiegebiet, um die elektrische Ankopplung zu verbessern. Hierdurch läßt sich der während der Koagulation auftretende Flüssigkeitsverlust ausgleichen, der sonst zu einer Änderung der elektrischen Impedanz des Gewebes im Therapiegebiet führt und die elektrische Ankopplung verschlechtert. Das Handhabungsteil 5 ermittelt deshalb aus der anliegenden Spannung und dem Strom über die Elektroden 3, 4 die Gewebeimpedanz und gibt eine entsprechende Menge Spülflüssigkeit an das Gewebe ab, um die Gewebeimpedanz konstant zuhalten. Die Spülflüssigkeit wird dem Handhabungsteil 5 über eine Schlauchleitung 13 von einer separaten Spülpumpe zugeführt und durch die hohle Mantelelektrode 4 ins Therapiegebiet gepumpt. Dort tritt die Spülflüssigkeit dann durch einen Spalt zwischen Kernelektrode 3 und Mantelelektrode 4 in das Gewebe aus.

Die Figuren 3a und 3b zeigen jeweils einen Katheter 14 bzw. 15, mit einer proximalen Elektrode 17 bzw. 19 und einer distalen Elektrode 16 bzw. 18, wobei der Abstand zwischen den beiden Elektroden 16, 17 bzw. 18, 19 konstant ist, um eine vorgegebene Feldverteilung zu erreichen und einen einfachen Aufbau des Katheters 14, 15 zu ermöglichen. Die beiden Elektroden 16, 17 bzw. 18, 19 sind hierbei zylindrisch ausgeführt und werden an ihren Stirnflächen durch ein ebenfalls zylindrisches Verbindungselement 20 bzw. 21 aus elektrisch isolierendem Material mechanisch mit einander verbunden, wobei das Verbindungselement 20 bzw. 21 wie auch die proximale Elektrode 17 bzw. 19 zur Aufnahme der Elektrodenzuleitung einen axial verlaufenden Hohlkanal aufweist. Bei dem in Figur 3a dargestellten Katheter 14 sind die Außenquerschnitte der beiden Elektroden 16, 17 und der Querschnitt des Verbindungselements 20 gleich, so daß die Außenkontur des Katheters 14 auch an den Übergangsstellen zwischen den Elektroden 16, 17 und dem Verbindungselement 20 glatt ist, wodurch das Einführen des Katheters 14 in den Körper des Patienten erleichtert wird. Bei dem in Figur 3b dargestellten Katheter 15 weist die proximale Elektrode 19 dagegen einen größeren Querschnitt auf als die distale Elektrode 18 und das Verbindungselement 21, wobei sich die proximale Elektrode 19 an der Übergangsstelle zu dem Verbindungselement 21 konisch auf den Querschnitt des Verbindungselements 21 verjüngt.

Figur 4 zeigt weiterhin einen Katheter 22, der sich von den vorstehend beschriebenen Kathetern im wesentlichen durch die größere Zahl von Elektroden 23.1 bis 23.5 unterscheidet sich, die entlang der Längsachse des Katheters 22 angeordnet sind und im wesentlichen aus ringförmigen metallischen Beschichtungen bestehen, die auf die Mantelfläche eines zylindrischen Trägerelements 24 aus elektrisch isolierendem Material aufgebracht sind. Die Kontaktierung der Elektroden 23.1 bis 23.5 erfolgt jeweils einzeln durch Zuleitungen, die in einem axial verlaufenden Hohlkanal des Trägerelements verlegt sind. Zum einen läßt sich durch die größere Zahl von Elektroden 23.1 bis 23.5 die partielle Stromdichte an den Elektroden 23.1 bis 23.5 reduzieren, wodurch ein zu großer Temperaturanstieg verhindert wird. Zum anderen läßt sich durch die Überlagerung der einzelnen Felder eine andere Feldverteilung erzeugen als bei nur zwei Elektroden. Darüber hinaus besteht die Möglichkeit, die Feldverteilung durch An- oder Abschaltung einzelner Elektrodenpaare gezielt zu beeinflussen.

Figur 5 zeigt als weiteres Ausführungsbeispiel der Erfindung einen Katheter 25, de flexibel ist und somit eine Einführung auch in Körperhöhlen mit gekrümmten Eingangskanälen, was insbesondere bei der minimalinvasiven Medizin (MIM) wichtig ist. Der Katheter 25 besteht im wesentlichen aus einer zylindrischen Kernelektrode 26 aus Federstahldraht, die von einer hohlzylindrischen Mantelelektrode 27 umgeben ist, welche aus einem flexiblen Metallgeflecht gebildet wird. Bis auf sein distales Ende ist die Kernelektrode 26 an seiner Mantelfläche mit einer elektrisch isolierenden Beschichtung 28 versehen, um die beiden Elektroden 26, 27 gegeneinander zu isolieren.

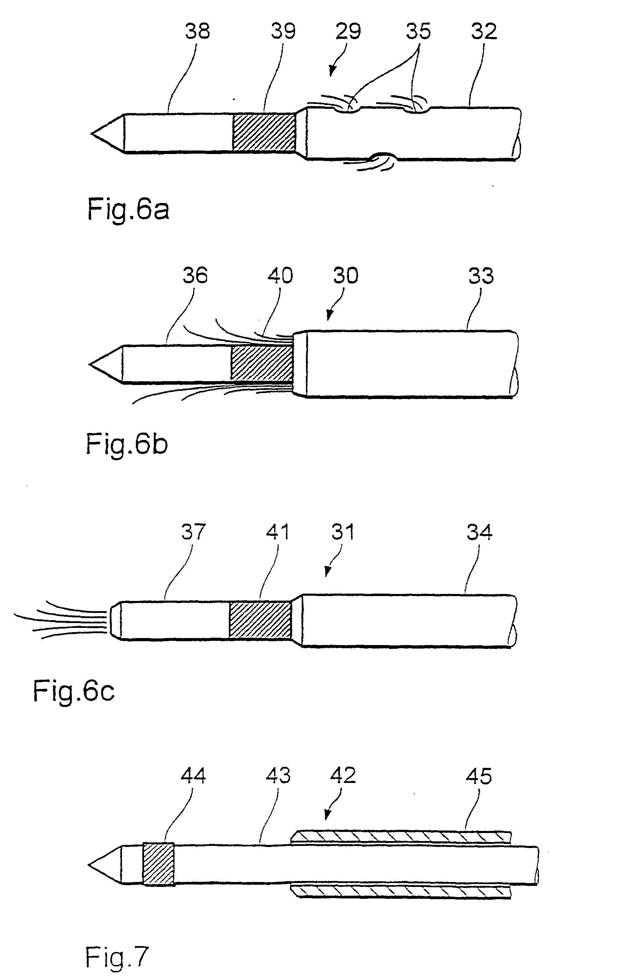

Die Figuren 6a, 6b und 6c zeigen weitere vorteilhafte Ausführungsformen von Kathetern 29, 30, 31 mit jeweils einer hohlzylindrischen proximalen Mantelelektrode 32, 33, 34 und einer zylindrisch ausgeführten distalen Kernelektrode 36, 37, 38. Die dargestellten Katheter 29, 30, 31 ermöglichen vorteilhaft eine Zuführung von Spülflüssigkeit in das Gewebe, um den Flüssigkeitsverlust des Gewebes während der Koagulation auszugleichen und eine damit verbundene Verschlechterung der elektrischen Ankopplung zu verhindern. Die Zuführung der Spülflüssigkeit erfolgt hierbei durch einen axial verlaufenden Hohlkanal in der proximalen Mantelelektrode 32, 33, 34 und wird durch eine extrakorporal angeordnete Spülmittelpumpe gewährleistet. Die Abgabe der Spülflüssigkeit im Therapiegebiet erfolgt bei den dargestellten Kathetern 29, 30, 31 dagegen in unterschiedlicher Weise. So weist der in Figur 6a dargestellte Katheter 29 in der Mantelfläche der Mantelelektrode 32 mehrere distal angeordnete Durchbrüche 35 auf, durch welche die Spülflüssigkeit aus dem Hohlkanal in das Gewebe austreten kann. Bei dem in Figur 6b dargestellten Katheter 30 tritt die Spülflüssigkeit dagegen durch einen Spalt zwischen Mantelelektrode 33 und Kernelektrode 36 in das Gewebe aus. Der in Figur 6c dargestellte Katheter 31 weist statt dessen einen in axialer Richtung durchgehenden Hohlkanal auf, der auch durch die Kernelektrode 37 hindurchläuft und in der distalen Stirnseite der Kernelektrode 37 mündet, so daß die Spülflüssigkeit an der distalen Stirnfläche der distalen Elektrode 37 in das Gewebe austritt.

Als Spülflüssigkeit wird vorzugsweise physiologische Kochsalzlösung verwendet, die für eine gute elektrische Ankopplung an das Gewebe sorgt und durch eine Begrenzung der Temperatur auf <100°C die Gefahr der Gewebekarbonisation vermindert. Die Isolation der beiden Elektroden 32, 38 bzw. 36, 33 bzw. 34, 37 gegeneinander erfolgt hierbei ebenfalls durch eine auf die Kernelektrode 36, 37, 38 aufgebrachte Beschichtung 39, 40, 41 aus elektrisch isolierendem Material.

Zusätzlich zur Zuführung von Spülflüssigkeit kann der Hohlkanal des in Figur 6c dargestellten Katheters 31 auch einen Lichtwellenleiter für eine modifizierte Optical Biopsy aufnehmen, der über eine Messung des Rückstreusignals oder der Gewebefluoreszenz bei Bestrahlung eine präzise Positionierung des Katheters 31 im Therapiegebiet ermöglicht. Darüber hinaus bietet eine Laserübertragung durch einen integrierten Lichtwellenleiter in Abhängigkeit von der verwendeten Wellenlänge auch die Möglichkeit einer Messung des Blutflusses durch Dopplermessung. Weiterhin kann die über einen derartigen Lichtwellenleiter ins Therapiegebiet übertragene Laserstrahlung auch zur thermo-optischen Gewebekoagulation verwendet werden. Schließlich besteht auch die Möglichkeit, durch den Hohlkanal einen Temperatursensor zur Koagulationssteuerung zu plazieren.

Figur 7 zeigt schließlich einen weiteren Katheter 42, der eine Einstellung des Elektrodenabstands ermöglicht, um die Feldverteilung im Therapiegebiet beeinflussen zu können. Hierzu weist der dargestellte Katheter 42 ein zylindrisches Trägerelement 43 aus elektrisch isolierendem Material auf, an dessem distalen Ende eine distale Elektrode 44 seitlich als ringförmige metallische Beschichtung aufgebracht ist.

Dieses Trägerelement 43 wird von einer hohlzylindrisch ausgeführten proximalen Elektrode 45 geführt, wobei der Außendurchmesser des Trägerelements 43 kleiner ist als der Innendurchmesser der proximalen Elektrode 45, so daß das Trägerelement 43 mit der distalen Elektrode 44 in axialer Richtung verschiebbar ist, um den Elektrodenabstand einstellen zu können. An seinem distalen Ende ist das Trägerelement 43 als Einstichdorn angeschliffen, um die Einführung des Katheters 42 in den Körper des Patienten zu erleichtern.

## Patentansprüche

1. Anordnung (1) zur elektro-thermischen Behandlung des menschlichen oder tierischen Körpers, insbesondere zur Gewebekoagulation oder Elektrotomie, mit zwei Elektroden (3, 4) zur Einführung in den zu behandelnden Körper, wobei die beiden Elektroden (3, 4) zur Erzeugung eines das Körpergewebe im Behandlungsgebiet erwärmenden elektrischen oder elektromagnetischen Feldes elektrisch gegeneinander isoliert und zueinander beabstandet angeordnet sowie über jeweils eine Zuleitung mit einer extrakorporal angeordneten Stromquelle verbunden sind, und wobei zur gemeinsamen Einführung der beiden Elektroden (3, 4) in den Körper ein langgestreckter Katheter (2) vorgesehen ist, auf dem die beiden Elektroden (3, 4) koaxial zueinander und in axialer Richtung des Katheters (2) gegeneinander versetzt angeordnet und mit dem Katheter (2) verbunden oder ein Bestandteil des Katheters (2) sind, und wobei die proximale Elektrode (4) zur Aufnahme der distalen Elektrode (3) mindestens auf einem distal gelegenen Teil ihrer Längserstreckung hohl ist, wobei der Außenquerschnitt der distalen Elektrode (3) derart an den Innenquerschnitt der proximalen Elektrode (4) angepasst ist, dass die beiden Elektroden (3, 4) zur Änderung des axialen Elektrodenbestandes und damit der Feldverteilung axial gegeneinander verschiebbar sind, und wobei die distale Elektrode (3, 16, 18, 26, 36, 37, 38) zur Erleichterung des Einführens in den Körper eine sich zu ihrem distalen Ende hin verjüngende Spitze aufweist **dadurch gekennzeichnet, dass** die proximale Elektrode (32, 33, 34) und/oder die distale Elektrode (37) zur Zuführung einer Spülflüssigkeit ins Therapiegebiet einen entlang ihrer Längsachse verlaufenden Hohlkanal aufweist, wobei zur Abgabe der Spülflüssigkeit im Therapiegebiet in der Wandung der proximalen Elektrode (32) mindestens ein Durchbruch (35) und/oder zwischen der proximalen Elektrode (33) und der distalen Elektrode (37) mindestens am distalen Ende der proximalen Elektrode (33) ein Spalt vorgesehen ist, der über die hohle proximale Elektrode (33) und ein einer Katheter hand habungsteil (5) mit einer an den Handhabungsteil (5) vorgesehene Schlauchleitung (13) für Spülflüssigkeit verbunden ist, die dem Handhabungsteil (5) über einen separaten Spülpumpe zu zuführen ist.

2. Anordnung nach Anspruch 1, **dadurch gekennzeichnet, dass** zur elektrischen Isolation der beiden Elektroden (3, 4) gegeneinander auf der Innenseite der proximalen Elektrode (4) und/oder auf der Außenseite der distalen Elektrode (3) eine bezüglich ihrer Längsachse umlaufende elektrische Isolierung (6) vorgesehen ist.

3. Anordnung nach Anspruch 1 oder 2, **dadurch gekennzeichnet, dass** die distale Elektrode (3) einen zylindrischen Außenquerschnitt aufweist und die proximale Elektrode (4) zur Aufnahme der distalen Elektrode (3) hohlzylindrisch ausgeführt ist.

4. Anordnung zur elektro-thermischen Behandlung des menschlichen oder tierischen Körpers, insbesondere zur Gewebekoagulation oder Elektrotomie, mit zwei Elektroden (3, 4) zur Einführung in den zu behandelnden Körper, wobei die beiden Elektroden (3, 4) zur Erzeugung eines das Körpergewebe im Behandlungsgebiet erwärmenden elektrischen oder elektromagnetischen Feldes elektrisch gegeneinander isoliert und zueinander beabstandet angeordnet sowie über jeweils eine Zuleitung mit einer extrakorporal angeordneten Stromquelle verbunden sind, und wobei zur gemeinsamen Einführung der beiden Elektroden (3, 4) in den Körper ein langgestreckter Katheter (2) vorgesehen ist, auf dem die beiden Elektroden (3, 4) koaxial zueinander und in axialer Richtung des Katheters (2) gegeneinander versetzt angeordnet und mit dem Katheter (2) verbunden oder ein Bestandteil des Katheters (2) sind, **dadurch gekennzeichnet, dass** der Katheter (42) ein langgestrecktes Trägerelement (43) aus elektrisch isolierendem Material aufweist, wobei die distale Elektrode (44) seitlich an dem Trägerelement (43) angebracht ist, und dass die proximale Elektrode (45) zur Aufnahme des Trägerelementes (43) mindestens auf einem distal gelegenen Teil ihrer Längserstreckung hohl ist und einen Innenquerschnitt aufweist, der derart an den Außenquerschnitt des Trägerelements (43) angepasst ist, dass das Trägerelement (43) mit der distalen Elektrode (44) und die proximale Elektrode (45) zur Änderung des axialen Elektrodenabstandes und damit der Feldverteilung axial gegeneinander verschiebbar sind, und wobei das Trägerelement (24, 43) zur Erleichterung des Einführens in den Körper eine sich zu ihrem distalen Ende hin verjüngende Spitze aufweist, und wobei die proximale Elektrode (32, 33, 34) und/oder die distale Elektrode (37) zur Zuführung einer Spülflüssigkeit ins Therapiegebiet einen entlang ihrer Längsachse verlaufenden Hohlkanal aufweist, und wobei zur Abgabe der Spülflüssigkeit im Therapiegebiet in der Wandung der proximalen Elektrode (32) mindestens ein Durchbruch (35) und/oder zwischen der proximalen Elektrode (33) und der distalen Elektrode (37) mindestens am distalen Ende der proximalen Elektrode (33) ein Spalt vorgesehen ist.

5. Anordnung nach Anspruch 4, **dadurch gekennzeichnet, dass** das Trägerelement (24, 43) einen zylindrischen Außenquerschnitt aufweist und mindestens eine Elektrode (23.1, 23.2, 23.3, 23.4, 44, 45) hohlzylindrisch ausgeführt und koaxial zu dem Trägerelement (24, 43) angeordnet ist.

6. Anordnung nach einem der vorhergehenden Ansprüche, **dadurch gekennzeichnet, dass** die proximale Elektrode (32, 33, 34) und/oder die distale Elektrode (37) zur Platzierung eines Temperatursensors oder eines Lichtwellenleiters einen entlang ihrer Längsachse verlaufenden Hohlkanal aufweist.

7. Anordnung nach einem der vorhergehenden Ansprüche, **dadurch gekennzeichnet, dass** die distale Elektrode im wesentlichen aus einer elektrisch leitfähigen Beschichtung auf der Mantelfläche eines Lichtwellenleiters besteht.

## Claims

1. An arrangement (1) for the electrothermal treatment of the human or animal boy, in particular for tissue coagulation or electronomy, having two electrodes (3, 4) for insertion into the body to be treated, wherein the two electrodes (3, 4) are electrically insulated from one another and are spaced from one another to produce an electric or electromagnetic field which heats the body tissue in the treatment area and also are each connected via a feed line with a current source provided outside the body, and wherein for the joint insertion of the two electrodes (3, 4) into the body an elongate catheter (2) is provided, on which the two electrodes (3, 4) are disposed coaxially to one another and staggered in relation to each other in the axial direction of the catheter (2) and are connected to the catheter (2) or are a component of the catheter (2), and wherein the proximal electrode (4) is hollow at least on a distally situated part of its longitudinal extension to house the distal electrode (3), whereby the outer cross section of the distal electrode (3) is adapted to the inner cross section of the proximal electrode (4) in such a manner that the two electrodes (3, 4) are axially displaceable in relation to one another to change the axial electrode spacing and thus the field distribution, and wherein the distal electrode (3, 16, 18, 26, 36, 37, 38) comprises a tip tapering towards its distal end to assist introduction into the body,
**characterised in that** the proximal electrode (32, 33, 34) and/or the distal electrode (37) comprises a hollow conduit running along its longitudinal axis for the supply of a rinsing liquid into the therapy region, wherein for the discharge of the rinsing liquid in the therapy region at least one opening (35) is provided in the wall of the proximal electrode and/or between the proximal electrode (33) and the distal electrode (37)a gap is provided at least at the distal end of the proximal electrode (33), which gap is connected via the hollow proximal electrode (33) and a catheter manipulator (5) with a hose line (13) for rinsing liquid provided at the manipulator (5), which liquid is to be supplied to the manipulator (5) via a separate rinsing pump.

2. An arrangement according to Claim 1,
**characterised in that** for the electrical insulation of the two electrodes (3, 4) from one another, on the inside of the proximal electrode (4) and/or on the outside of the distal electrode (3) there is provided an electrical insulation (6) that extends circumferentially in relation to the longitudinal axis.

3. An arrangement according to Claim 1 or 2,
**characterised in that** the distal electrode (3) comprises a cylindrical outer cross section and the proximal electrode (4) has a hollow cylindrical construction to receive the distal electrode (3).

4. An arrangement for the electrothermal treatment of the human or animal body, in particular for tissue coagulation or electronomy, having two electrodes (3, 4) for insertion into the body to be treated, wherein the two electrodes (3, 4) are electrically insulated from one another to produce an electric or electromagnetic field which heats the body tissue in the treatment area and are spaced from one another and also are each connected via a feed line to a current source provided outside the body, and wherein for the joint insertion of the two electrodes (3, 4) into the body an elongate catheter (2) is provided, on which the two electrodes (3, 4) are disposed coaxially in relation to one another and staggered in relation to each other in the axial direction of the catheter (2) and are connected to the catheter (2) or are a component of the catheter,
**characterised in that** the catheter (42) is an elongate carrier element (43) made from electrically insulating material, the distal electrode (44) being mounted laterally on the carrier element (43),
and **in that** the proximal electrode (45) is hollow to house the carrier element (43) at least one a distally positioned part of its longitudinal extension and comprises an inner cross section which is adapted to the outer cross section of the carrier element (43) in such a manner that the carrier element (43) with the distal electrode (44) and the proximal electrode (45) are axially displaceable in relation to each other to change the axial electrode spacing and thus the field distribution, and wherein the carrier element (24, 43) comprises a tip tapering towards its distal end to assist introduction into the body, and wherein the proximal electrode (32, 33, 35) and/or the distal electrode (37) comprises a hollow conduit running along its longitudinal axis to supply a rinsing liquid into the therapy area, and wherein to discharge the rinsing liquid in the therapy region at least one opening (35) is provided in the wall of the proximal electrode (32) and/or between the proximal electrode (33) and the distal electrode (37) a gap is provided at least at the distal end of the proximal electrode (33).

5. An arrangement according to Claim 4,
**characterised in that** the carrier element (24, 43) comprises a cylindrical outer cross section and at least one electrode (23.1, 23.2, 23.3, 23.4, 44, 45) has a hollow cylindrical construction and is disposed coaxially to the carrier element (24, 43).

6. An arrangement according to one of the preceding Claims,
**characterised in that** the proximal electrode (32, 33, 34) and/or the distal electrode (37) comprises a hollow conduit extending along its longitudinal axis for positioning a temperature sensor or an optical waveguide.

7. An arrangement according to one of the preceding Claims,
**characterised in that** the distal electrode essentially consists of an electrically conductive coating on the surface of an optical waveguide.

## Revendications

1. Système (1) pour le traitement électrothermique du corps humain ou animal, en particulier pour la coagulation de tissu ou l'électrotomie, comportant deux électrodes (3, 4) destinées à être introduites dans le corps à traiter, où les deux électrodes (3, 4) sont isolées électriquement l'une par rapport à l'autre pour la production d'un champ électrique ou électromagnétique chauffant le tissu corporel dans le domaine du traitement et sont disposées à distance l'une de l'autre et sont reliées chacune par un conducteur d'alimentation à une source de courant disposée extracorporellement et où pour l'introduction commune des deux électrodes (3, 4) dans le corps, il est prévu un cathéter allongé (2) sur lequel les deux électrodes (3, 4) sont disposées coaxialement l'une par rapport à l'autre et décalées l'une par rapport à l'autre dans la direction axiale du cathéter (2) et sont reliées au cathéter (2) ou sont un constituant du cathéter (2), et où l'électrode proximale (4) est creuse au moins sur une partie disposée distalement de son étendue longitudinale pour recevoir l'électrode distale (3), où la section transversale externe de l'électrode distale (3) est adaptée à la section transversale interne de l'électrode proximale (4) de telle manière que les deux électrodes (3, 4) peuvent être déplacées axialement l'une par rapport à l'autre pour modifier la distance axiale entre les électrodes et donc la répartition du champ, et où l'électrode distale (3, 16, 18, 26, 36, 37, 38) comporte une pointe qui s'amincit en direction de son extrémité distale pour faciliter l'introduction dans le corps, **caractérisé en ce que** l'électrode proximale (32, 33, 34) et/ou l'électrode distale (37) comportent un canal creux qui s'étend le long de leur axe longitudinal pour l'introduction d'un liquide de lavage dans le domaine de la thérapie, où, pour délivrer le liquide de lavage dans le domaine de la thérapie, il est prévu dans la paroi de l'extrémité proximale (32) au moins une ouverture (35) et/ou entre l'électrode proximale (33) et l'électrode distale (37) au moins à l'extrémité distale de l'électrode proximale (33) une fente qui est reliée par l'intermédiaire de l'électrode proximale creuse (33) et d'une partie de maniement de cathéter (5) avec une conduite de type tuyau (13) qui est prévue sur la partie de maniement (5) pour le liquide de lavage qui doit être envoyé à la partie de maniement (5) par le biais d'une pompe de lavage séparée.

2. Système selon la revendication 1 **caractérisé en ce que**, pour l'isolation électrique des deux électrodes (3, 4) l'une par rapport à l'autre, il est prévu sur le côté interne de l'électrode proximale (4) et/ou sur le côté externe de l'électrode distale (3) une isolation électrique (6) périphérique par rapport à leur axe longitudinal.

3. Système selon la revendication 1 ou 2 **caractérisé en ce que** l'électrode distale (3) présente une section transversale externe cylindrique et l'électrode proximale (4) est cylindrique creuse pour recevoir l'électrode distale (3).

4. Système pour le traitement électrothermique du corps humain ou animal, en particulier pour la coagulation de tissu ou l'électrotomie, comportant deux électrodes (3, 4) destinées à être introduites dans le corps à traiter, où les deux électrodes (3, 4) sont isolées électriquement l'une par rapport à l'autre pour la production d'un champ électrique ou électromagnétique chauffant le tissu corporel dans le domaine du traitement et sont disposées à distance l'une de l'autre et sont reliées chacune par le biais d'un conducteur d'alimentation à une source de courant disposée extracorporellement, et où, pour l'introduction commune des deux électrodes (3, 4) dans le corps, il est prévu un cathéter allongé (2) sur lequel les deux électrodes (3, 4) sont disposées coaxialement l'une par rapport à l'autre et décalées l'une par rapport à l'autre dans la direction axiale du cathéter (2) et sont reliées au cathéter (2) ou sont un constituant du cathéter (2), **caractérisé en ce que** le cathéter (42) comporte un élément de support allongé (43) en matériau électriquement isolant, où l'électrode distale (44) est disposée latéralement sur l'élément de support (43), et **en ce que** l'électrode proximale (45) est creuse au moins sur une partie disposée distalement de son étendue longitudinale pour recevoir l'élément de support (43) et présente une section transversale interne qui est adaptée à la section transversale externe de l'élément de support (43) de telle manière que l'élément de support (43) comportant l'électrode distale (44) et l'électrode proximale (45) sont déplaçables axialement mutuellement pour modifier la distance axiale entre les électrodes et donc la répartition du champ, et où l'élément de support (24, 43) comporte une pointe qui s'amincit en direction de son extrémité distale pour faciliter l'introduction dans le corps, et où l'électrode proximale (32, 33, 34) et/ou l'électrode distale (37) comportent un canal creux qui s'étend le long de leur axe longitudinal pour l'introduction d'un liquide de lavage dans le domaine de la thérapie, et où, pour la délivrance du liquide de lavage dans le domaine de la thérapie, il est prévu dans la paroi de l'électrode proximale (32) au moins une ouverture (35) et/ou entre l'électrode proximale (33) et l'électrode distale (37), au moins à l'extrémité distale de l'électrode proximale (33), une fente.

5. Système selon la revendication 4 **caractérisé en ce que** l'élément de support (24, 43) comporte une section transversale externe cylindrique et au moins une électrode (23.1, 23.2, 23.3, 23.4, 44, 45) est cylindrique creuse et disposée coaxialement à l'élément de support (24, 43).

6. Système selon l'une des revendications précédentes **caractérisé en ce que** l'électrode proximale (32, 33, 34) et/ou l'électrode distale (37) comportent un canal creux qui s'étend le long de leur axe longitudinal pour la mise en place d'un capteur de température ou d'un guide d'ondes lumineuses.

7. Système selon l'une des revendications précédentes **caractérisé en ce que** l'électrode distale consiste essentiellement en un revêtement électriquement conducteur sur la surface d'enveloppe d'un guide d'ondes lumineuses.
